# EUROPEAN PATENT APPLICATION

(11) **EP 4 599 842 A1**
(43) Date of publication of application: **13.08.2025**
(21) Application number: 24305219.8
(22) Date of filing: 09.02.2024
(51) Int. Cl.: A61K 38/18, C07K 14/475

(54) **PEGYLATED GDF5 PROTEIN**

(71) Applicant: Association Institut de Myologie, 75013 Paris (FR); Institut National de la Santé et de la Recherche Médicale, 75013 Paris (FR); SORBONNE UNIVERSITE, 75006 Paris (FR)
(72) Inventor: PIETRI-ROUXEL, France, CLICHY LA GARENNE (FR); FALCONE, Sestina, PARIS (FR); NOVIELLO, Chiara, PARIS (FR); TRAORE, Massiré, PARIS (FR)
(74) Representative: Cabinet Becker et Associés

(57) **Abstract**

The present invention relates to a pegylated GDFS protein and uses thereof.

## Description

### FIELD OF THE INVENTION

The present invention relates to a pegylated GDF5 protein and uses thereof.

### BACKGROUND OF THE INVENTION

Growth Differentiation Factor 5 (GDF5) is a blood circulating factor, expressed by skeletal muscle after nerve damage, playing a critical role in limiting muscle atrophy (Sartori et al. Nat Genet., 2013, 45, 1309-1318). Our previous work demonstrated that GDF5 overexpression, achieved by intramuscular injection of an adenoassociated virus (AAV), is also able to preserve muscle mass in aged muscles (Traoré et al., Sci Transl Med., 2019, Nov 6; 11(517)). Following these results, we developed a chronic treatment of sarcopenia based on the systemic administration of recombinant GDF5 protein, and observed that this treatment preserved muscle mass and function during aging. These outstanding results led us to search to optimize human GDF5 to further improve its clinical use.

### SUMMARY OF THE INVENTION

It is herein described a pegylated GDF5 (GDF5-PEG) protein.

In a particular embodiment, the GDF5-PEG protein is a recombinant or chemically synthesized GDF5 protein, in particular a chemically synthesized GDF5 protein.

In another particular embodiment, the GDF5-PEG protein is a human GDF5 protein. In yet another embodiment, the GDF5-PEG protein is a recombinant or chemically synthesized human GDF5 protein, in particular a chemically synthesized human GDF5 protein. In a particular embodiment, the amino acid sequence of the mature human GDF5 protein may comprise or consist of the amino acid sequence shown in SEQ ID NO:2 or 3, or may comprise or consist of a sequence which is at least 90% identical to SEQ ID NO:2 or 3, in particular at least 91%, at least 92%, at least 93%, at least 94%, at least 95%, at least 96%, at least 97%, at least 98% or at least 99% identical to SEQ ID NO:2 or 3.

In another embodiment, the GDF5-PEG protein comprises a PEG molecule having a molecular weight of from 5,000 g/mol to 50,000 g/mol. In a particular embodiment, the PEG is selected from linear or branched PEG, in particular linear PEG. In a further particular embodiment, the PEG molecule has a molecular weight of from 10,000 to 30,000 g/mol, in particular from 15,000 to 25,000 g/mol, such as from 16,000 to 24,000 g/mol, in particular from 17,000 to 23,000 g/mol, more particularly from 18,000 to 22,000 g/mol, even more particularly from 19,000 to 21,000 g/mol. In yet another embodiment, the PEG molecule has a molecular weight of 20,000 g/mol.

In another aspect, it is described the use of the GDF5-PEG disclosed herein in therapy. In some embodiments, the GDF5-PEG protein is used in a method for the treatment of sarcopenia or disuse atrophy. In other embodiments, the GDF5-PEG is used in a method for treating or preventing muscle weakness in a myopathy or neuromuscular disease, alone or in combination with a treatment of said myopathy or neuromuscular disease. In yet other aspects, the GDF5-PEG protein is used in the treatment of a muscular disease, in combination with at least one other active ingredient suitable for the treatment of said muscular disease.

### DESCRIPTION OF THE DRAWINGS

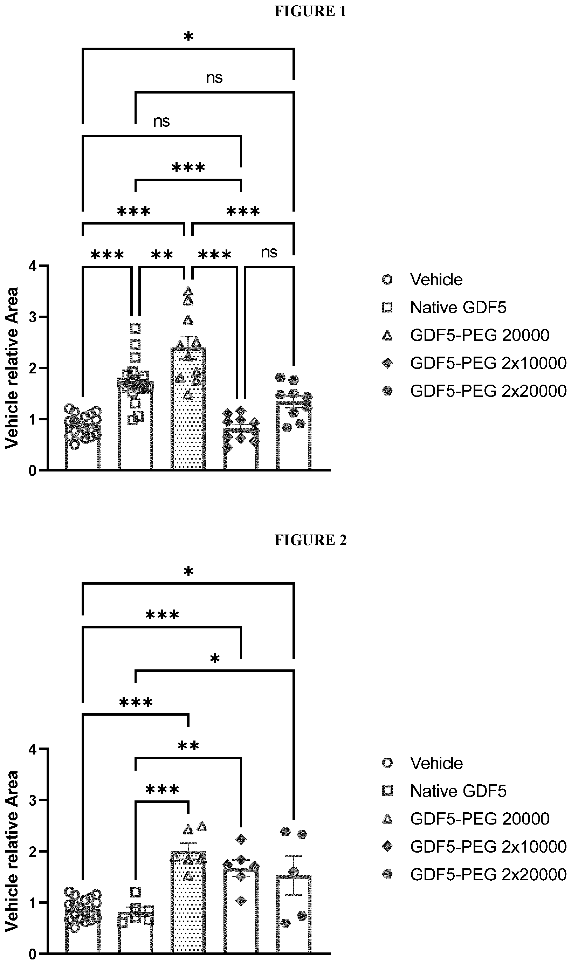

**Figure 1****:** Measure of the intensity of p-SMAD1/5-positive nuclei 48 h after injection of native GDF5 or various pegylated forms of GDF5. Tibialis Anterior (TA) muscle sections were immuno-fluorescently labelled with anti-p-SMAD1/5 antibody. Signal intensity of the nuclei within the slice was measured with an ImageJ software script and analyzed in Excel with a normal distribution. The area under the curve was measured and plotted as relative area to the vehicle. ANOVA 1 factor and Tukey HSD P-value : * <0,05 ; **<0,01 ; ***< 0,001. Vehicle: n=10 mice, Native GDF5: n=6 mice; GDFS-PEG 20,000: n=5 mice; GDF5-PEG 2x10,000: n=5 mice; GDF5-PEG 2x20,000 : n=5 mice.
**Figure 2****:** Measure of the intensity of p-SMAD1/5 positive nuclei 7 days after injection of native GDF5 and various pegylated forms of GDF5. The area under the curve was measured and plotted as relative area versus vehicle. ANOVA 1 factor and ANOVA 1 factor and Tukey HSD P-value : * <0,05 ; **<0,01 ; ***< 0,001. Vehicle : n=10 mice, Native GDF5: n=3 mice; GDF5-PEG20,000 : n=3 mice ; GDF5-PEG 2x10,000 : n=3 mice; GDF5-PEG 2x20,000: n=3 mice.

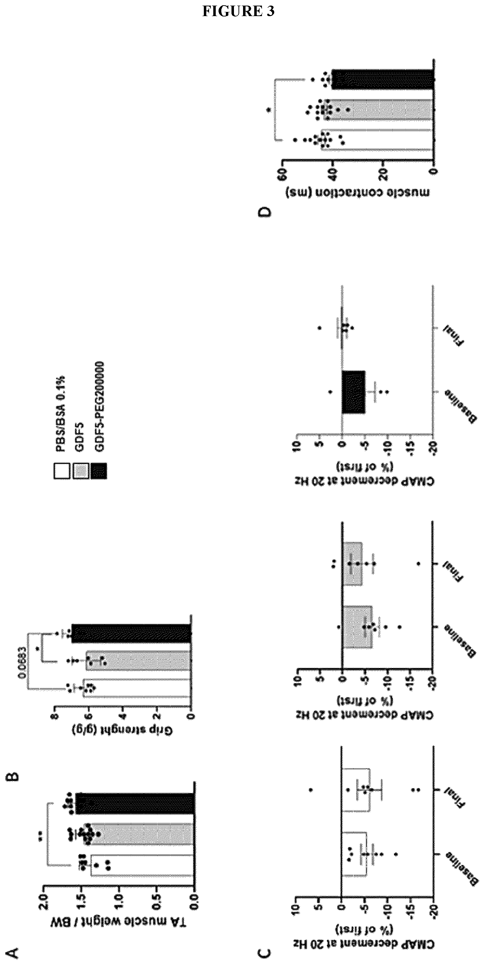

**Figure 3****:** Functional benefits of treatment with GDF5-PEG20,000. Injections were made twice weekly subcutaneously for 16 weeks with vehicle solution (PBS-0.1% BSA) (white) or native human GDF5 (light gray) or GDF5-PEG20,000 (dark gray) at a dose of 0.2 mg/kg. All measurements were made at the end of the treatment.

A. Ratio of TAs weight to overall mouse weight.
B. Ratio of grip force to mouse weight, this measurement takes into account the force produced by the muscles of all four limbs.
C. Decrement of TAs muscle action potential between the first and tenth depolarization following sciatic nerve stimulation at a frequency of 20 Hz. The final value (Final) measured at the end of the protocol after 4 months of injections is compared with the value measured before the start of injections (Baseline).
D. Muscle contraction time (ms) to reach maximal force measured after electrical stimulation of mice sciatic nerves at a frequency of 75-150 Hz

A, B and D: 1-factor ANOVA Tukey's HSD or Fisher's LSD tests; C: T-test. P-value : * <0,05 ; **<0,01 ; ***< 0,001. PBS/BSA 0.1% n= 4 mice; Native GDF5 n=7 mice; GDF5-PEG 20,000 n=7 mice.

### DETAILED DESCRIPTION OF THE INVENTION

### GDF5-PEG protein

In the context of the present disclosure, the "percentage identity" between two sequences (A) and (B) is determined by comparing the two sequences aligned in an optimal manner, through a window of comparison. The percent identity between the two sequences is particularly a function of the number of identical positions shared by the sequences (i.e., % identity = number of identical positions/total number of positions x 100), taking into account the number of gaps, and the length of each gap, which need to be introduced for optimal alignment of the two sequences. Said alignment of sequences can be carried out by well-known methods in the art, for example, using the algorithm for global alignment of Needleman-Wunsch. Protein analysis software matches similar sequences using measures of similarity assigned to various substitutions, deletions, and other modifications, including conservative amino acid substitutions. Once the total alignment is obtained, the percentage of identity can be obtained by dividing the full number of identical amino acid residues aligned by the full number of residues contained in the longest sequence between the sequence (A) and (B). Sequence identity is typically determined using sequence analysis software. For comparing two amino acid sequences, one can use, for example, the tool "Emboss needle" for pairwise sequence alignment of proteins providing by EMBL-EBI and available on:
www.ebi.ac.uk/Tools/services/web/toolform.ebi?tool=emboss_needle&context=protein, for example using default settings: (I) Matrix: BLOSUM62, (ii) Gap open: 10, (iii) gap extend: 0.5, (iv) output format: pair, (v) end gap penalty: false, (vi) end gap open: 10, (vii) end gap extend: 0.5.

"Native amino acid sequence" or "native protein" is to be understood according to the skilled person's general understanding in the art and denotes the amino acid sequence in the form of its occurrence in nature without any mutation or amino acid modification made by a human. It can also be referred to as a "wild-type sequence". Thus, "native GDF5", "native GDF5 amino acid sequence", "wild-type GDF5" or "wild-type GDF5 amino acid sequence" denotes mature GDF5 having the amino acid sequence as it occurs in nature, such as the mature human GDF5 as shown in SEQ ID NO: 2 or 3. The presence or absence of an N-terminal methionine, which depends on the used expression host, if any, usually does not change the status of a protein being considered as having its natural or native/wild-type sequence.

The present disclosure is based on the discovery that pegylated GDF5 (GDF5-PEG) has improved properties over native, non-pegylated GDF5 protein. Accordingly, it is herein described a GDF5-PEG protein. Such a GDF-PEG protein comprises a poly(ethyleneglycol) (PEG) moiety attached to a GDF5 protein moiety.

Unprocessed wild-type human GDF-5 (Uniprot Accession No. P43026) has the following sequence:

MRLPKLLTFLLWYLAWLDLEFICTVLGAPDLGQRPQGTRPGLAKAEAKERPPLARNVFRPGG
HSYGGGATNANARAKGGTGQTGGLTQPKKDEPKKLPPRPGGPEPKPGHPPQTRQATARTVTP
KGQLPGGKAPPKAGSVPSSFLLKKAREPGPPREPKEPFRPPPITPHEYMLSLYRTLSDADRKGG
NSSVKLEAGLANTITSFIDKGQDDRGPVVRKQRYVFDISALEKDGLLGAELRILRKKPSDTAKP
AAPGGGRAAQLKLSSCPSGRQPASLLDVRSVPGLDGSGWEVFDIWKLFRNFKNSAQLCLELE
AWERGRAVDLRGLGFDRAARQVHEKALFLVFGRTKKRDLFFNEIKARSGQDDKTVYEYLFSQ
RRKRRAPLATRQGKRPSKNLKARCSRKALHVNFKDMGWDDWIIAPLEYEAFHCEGLCEFPLR
SHLEPTNHAVIQTLMNSMDPESTPPTCCVPTRLSPISILFIDSANNVVYKQYEDMVVESCGCR
(SEQ ID NO:1)

SEQ ID NO:1 comprises a signal peptide at amino acid positions 1-27, a propeptide at amino acid positions 28-381 and a part, underlined in the sequence provided above, corresponding to the mature peptide at amino acid positions 382-501.

The mature peptide thus has a sequence as shown in SEQ ID NO:2 below:

APLATRQGKRPSKNLKARCSRKALHVNFKDMGWDDWIIAPLEYEAFHCEGLCEFPLRSHLEP
TNHAVIQTLMNSMDPESTPPTCCVPTRLSPISILFIDSANNVVYKQYEDMVVESCGCR
(SEQ ID NO:2).

Other human mature GDF5 proteins are commercially available, such as the protein having the sequence shown in SEQ ID NO:3, which is available from Thermo Fischer (catalog No. RP-8663):

APSATRQGKRPSKNLKARCSRKALHVNFKDMGWDDWIIAPLEYEAFHCEGLCEFPLRSHLEP
TNHAVIQTLMNSMDPESTPPTCCVPTRLSPISILFIDSANNVVYKQYEDMVVESCGCR
(SEQ ID NO:3)

In the context of the present disclosure, the proteins shown in SEQ ID NO:2 and 3 may be referred to as "mature GDF5" or declinations thereof.

In some embodiments, the GDF5 protein moiety is a functional derivative of a mature GDF5 protein. A functional derivative according to this disclosure is a protein having at least one, in particular all, activity of a reference protein. In the context of the present disclosure, a functional variant of a GDF5 protein may have its ability to induce alkaline phosphatase production by ATDC5 mouse chondrogenic cells (Nakamura, K. et al. (1999) Exp. Cell Res. 250:351) with an ED50 from 0.01 to 10 µg/mL, such as from 0.2 to 4 µg/mL, for example from 0.2 to 1.2 µg/mL. In particular, a functional variant of a GDF5 protein is a protein that may treat or prevent sarcopenia in an animal model of the condition or in a human subject. GDF5 signaling may also be evaluated by measuring SMAD 1/5/8 phosphorylation, SMAD 4 nuclear translocation and Id-1 transcription as provided in the experimental part below. The activity of the functional variant may be of at least 30%, 35%, 40%, 45%, 50%, 55%, 60%, 65%, 70%, 75%, 80%, 85%, 90%, 91%, 92%, 93%, 94%, 95%, 96%, 97%, 98%, 99% or at least 100% of the activity of the reference GDF5 protein. In some embodiments, the functional peptide as an activity greater than the activity of the reference GDF5 peptide, such as an activity of at least 105%, 110%, 115%, 120%, 125%, 130%, 135%, 140%, 145%, or of at least 150% of the activity of the reference GDF5 peptide. In addition, according to the present disclosure, a functional variant of a GDF5 protein has at least 90% sequence identity to a reference GDF5 amino acid sequence, in particular at least 91%, 92%, 93%, 94%, 95%, 96%, 97%, 98% or at least 99% sequence identity to a reference mature human GDF5. For example, a functional variant of a GDF5 protein may comprise from 1 to 12 amino acid modifications (i.e. amino acid addition, deletion or substitution) as compared to a reference mature human GDF5, such as from 1 to 11 amino acid modifications, in particular from 1 to 10, 1 to 9, 1 to 8, 1 to 7, 1 to 6, 1 to 5, 1 to 4, 1 to 3, 1 to 2 or one amino acid modification(s), as compared to a reference mature human GDF5. Such a functional variant of mature human GDF5 may be a natural variant of GDF5. In a particular aspect, the functional variant is an optimized GDF5 protein. Optimization may include different changes in the peptide, such as amino acid modifications as provided above, glycosylation, acetylation, phosphorylation and the like, or inclusion of at least one D-amino acid, such as at least 2, at least 3, at least 4 or at least 5 D amino acids. In another aspect, the GDF5 protein moiety comprises at least one non-natural amino acid, included by insertion, appendage, or substitution for another amino acid of the GDF5 sequence. In yet another aspect, recombinant GDF5 may be fused to another moiety, such as another peptide moiety. Such other moiety may, for example, stabilize the peptide.

In another embodiment, the GDF5 protein moiety is a functional variant of a GDF5 protein corresponding to the GDF5-related proteins as described in WO201308649, having an increased affinity for the BMP receptor IB (BMPR-IB) and/or a reduced affinity for the BMP receptor IA (BMPR-IA). In some embodiments, the protein is derived from human wild-type GDF5. In a particular embodiment, the GDF5-related protein is obtained by replacing at least one amino acid residue relating to a BMPR-IB and/or BMPR-IA binding site in the amino acid sequence of the GDF-5 protein, preferably by genetic engineering technology. In a further embodiment, at least one hydrophobic amino acid in the BMPR-IB and/or BMPR-IA binding site the GDF5 protein is replaced with a hydrophilic or polar amino acid, such as a hydrophilic amino acid residue or polar amino acid residue selected from the group consisting of aspartic acid, glutamic acid, lysine, arginine, histidine, serine and threonine. In an alternative embodiment, at least one hydrophilic or polar amino acid in the BMPR-IB and/or BMPR-IA binding site of the GDF5 protein is replaced with a hydrophobic amino acid, such as a hydrophobic amino acid selected from the group consisting of alanine, isoleucine, leucine, methionine, phenylalanine, proline, tryptophan, tyrosine, valine. In another alternative embodiment, the GDF5-related protein comprises a conservative substitution of at least one amino acid in the BMPR-IB and/or BMPR-IA binding site of the GDF protein, in particular wherein a hydrophobic amino acid is replaced by a smaller or larger hydrophobic amino acid or wherein a hydrophilic or polar amino acid is replaced by a smaller or lager hydrophilic or polar amino acid. The regions of GDF-5 related proteins which are involved in binding to BMPR-IA and/or BMPR-IB are well known in the art or can easily be determined using methods that are within common knowledge. Referring to the unprocessed, full-length amino acid sequence of wild-type human GDF5 of SEQ ID NO: 1, a particular embodiment provides the replacement of one or more of the following amino acids by any different amino acid:
R399;
any one of F409 to W417, in particular M412, G413, W414, and/or W417;
any one of E 434 to M 456, in particular F435, P436, L437, R438, S439, H440, P443, N445, V448, I449, L452, M453, S455, and/or M456;
S475;
I476;
F478;
any one of K488 to M493, in particular K488, Y490, and/or D492.

In particular embodiments, referring to the unprocessed, full-length amino acid sequence of SEQ ID NO: 1, one or more of the following amino acids are replaced by the specified amino acid
- R399 is replaced by V, L, I, M, F, Y, W, E or D;
- M412 is replaced by V, L, I, F, Y, W, H, K or R;
- W414 is replaced by R, K, F, Y, H, E or D;
- W417 is replaced by R, K, F, Y, H, E or D;
- F435 is replaced by V, L, I, M, P, Y, W, H, K or R;
- P436 is replaced by V, L, I, M, F, Y or W;
- L437 is replaced by D or E;
- R438 is replaced by K, D, H, N, M, E, Q, S, T, Y or W;
- S439 is replaced by K, D, E, H, R, M, T, N, Q, Y or W;
- H440 is replaced by V, I, M, F, Y, W, E or D;
- P443 is replaced by V, L, I, M, F, Y, W, A or S;
- N445 is replaced by D, Q, H, F, L, R, K, M, S, Y or W;
- V448 is replaced by F, L, I, M, P, Y or W;
- I449 is replaced by F, L, V, M, P, Y or W;
- L452 is replaced by F, I, V, M, P, Y or W;
- M456 is replaced by F, I, L, P, Y, W, S, T, N, Q, K or D;
- S475 is replaced by M, T, N, Q, Y or W;
- K488 is replaced by R, M, S, T, N, Q, Y or W;
- Y490 is replaced by E, H, K, R, Q, F, T, M, S, N, Q or W;
- D492 is replaced by G, E, M, S, T, N, Q, Y, W, H, K or R;
- I476 is replaced by G, A, V, L, M, F, Y or W;
- F478 is replaced by G, A, V, L, I, Y or W.

In another particular embodiment, referring to the unprocessed, full-length amino acid sequence of SEQ ID NO: 1, one or more of the following amino acids are replaced by the specified amino acid:
R399 is replaced by M or E;
W414 is replaced by R;
W417 is replaced by R or F;
R438 is replaced by K;
S439 is replaced by K or E;
I449 is replaced by V.

The corresponding positions in the mature peptides (such as in SEQ ID NO:2 or SEQ ID NO:3) will easily be derived from the above information regarding unprocessed, full-length wild-type human GDF5.

In some embodiments, the GDF5 protein moiety can comprise or consist of the amino acid sequence of a mature native GDF5 protein, or can comprise or consist of an amino acid sequence which is at least 90%, at least 91%, at least 92%, at least 93%, at least 94%, at least 95%, at least 96%, at least 97%, at least 98% or at least 99% identical to a mature native GDF5 protein. Illustrative mature native GDF5 proteins include, without limitation, GDF5 proteins of any genus or species, such as GDF5 of mammalian origin. In a particular embodiment, the GDF5 protein is human GDF5. In an embodiment, the human GDF5 protein moiety of the GDF5-PEG protein moiety can comprise or consist of the amino acid sequence shown in SEQ ID NO:2 or 3, or can comprise or consist of a sequence which is at least 90% identical to SEQ ID NO:2 or 3, in particular at least 91%, at least 92%, at least 93%, at least 94%, at least 95%, at least 96%, at least 97%, at least 98% or at least 99% identical to SEQ ID NO:2 or 3.

According to the present disclosure, the GDF5 protein can be a recombinantly expressed or chemically synthesized GDF5 protein, in particular a chemically synthesized GDF5 protein.

Recombinant expression can be achieved using recombinant DNA techniques. Such techniques involve the introduction of the transgene encoding the protein of interest into a host cell suitable for expression of the transgene, and recovery of the expressed GDF5 protein.

Chemical synthesis of a protein refers to cell-free protein synthesis. In some embodiments, the synthesis of the GDF5 protein moiety comprises ligating two or more chemically synthesized fragments of the GFD5 protein moiety, such as three or more fragments, for examples four fragments. Chemical synthesis of a protein or fragments thereof can be carried with different methods, such as by solid phase peptide synthesis (SPPS) or solution phase synthesis (SPS). GDF5 protein moiety is synthesized on an automated synthesizer.

In some embodiments, the synthetic GDF5 protein moiety is prepared from one or more chemically synthesized fragments. In some embodiments, the synthetic GDF5 protein moiety is prepared from 1, 2, 3, 4, 5, 6, 7, 8, or more chemically synthesized fragments. In some embodiments, the synthetic I GDF5 protein moiety is prepared from 3, 4 or 5 chemically synthesized fragments. In some embodiments, the synthetic GDF5 protein moiety is prepared from 4 chemically synthesized fragments.

An illustrative, non-limiting, synthetic scheme of a GDF5 protein moiety modified with a polymer can include the following method. In some embodiments, a first ("Part 1"), second ("Part 2"), third ("Part 3") and fourth ("Part 4") fragments corresponding to consecutive amino acid residues of the GDF5 protein are synthesized separately, for example by SPPS or SPS. The first fragment is assembled with the second fragment to produce a single fragment ("Part 12"). The third fragment is then assembled with the Part 12 fragment to produce a longer single fragment ("Part 123"). The fourth fragment is then is then assembled with Part 123 to produce the full-length fragment ("Part 1234"). The full-length fragment is then folded to produce the GDF5 protein.

In some embodiments, the PEG molecule is covalently attached to one or more amino acid residue(s) of the assembled full-length GDF5 protein moiety. In other embodiments, before assembly into the full-length GDF5 protein moiety, one or more fragments are modified with a PEG molecule covalently attached to one or more amino acid residue(s) comprised in said fragment(s).

As mentioned above, the GDF5-PEG protein is produced by covalently attaching a PEG molecule to the GDF5 protein moiety. In some embodiments, the PEG is selected from linear or branched PEG, in particular linear PEG. In some embodiments, the GDF5-PEG protein comprises a linear PEG molecule having a molecular weight of from 5,000 g/mol to 50,000 g/mol. In a further particular embodiment, the linear PEG molecule has a molecular weight of from 10,000 to 30,000 g/mol, in particular from 15,000 to 25,000 g/mol, such as from 16,000 to 24,000 g/mol, in particular from 17,000 to 23,000 g/mol, more particularly from 18,000 to 22,000 g/mol, even more particularly from 19,000 to 21,000 g/mol. In yet another embodiment, the linear PEG molecule has a molecular weight of 20,000 g/mol.

### Uses of the GDF5-PEG protein

In the context of the present disclosure, the terms "subject" and "patient" are used interchangeably and refer to a mammal being assessed for treatment or being treated. Subjects may be mammals, in particular human.

The term "treatment" or "treating" refers to an action, application or therapy, wherein a subject, in particular a human being, is subjected to medical aid with the purpose of improving the subject's condition, directly or indirectly. Particularly, the term refers to reducing incidence, or alleviating symptoms, eliminating recurrence, preventing recurrence, preventing incidence, improving symptoms, improving prognosis or combination thereof in some embodiments. The skilled artisan understands that treatment does not necessarily result in the complete absence or removal of symptoms.

Thanks to the advantageous properties of the GDF5-PEG protein shown in the experimental section below, several important therapeutic and non-therapeutic uses of said protein can be envisioned. For example, one skilled in the art can use the GDF5-PEG protein of the present disclosure as an improved alternative to the GDF5 protein in the uses disclosed in WO2020016425 and WO 2021/089736, which are incorporated by reference.

In a particular embodiment, the GDF5-PEG protein is used for the treatment of sarcopenia or disuse atrophy.

Sarcopenia is an age-related condition characterized by a progressive loss of skeletal muscle mass and function. From age 50, muscle quantity and strength start to decrease and typically, more than 30% of muscle initial mass is lost at 80 years old. Sarcopenia is a major clinical problem in public health of older people, with adverse outcomes such as disability, poor quality of life, hospitalization needs and increased risk of death. Sarcopenia may lead to frailty and several studies have shown that the risk of falls is significantly elevated in subjects with reduced muscle strength. This condition raises major concerns, and it is important to prevent or postpone as much as possible the onset of this condition, to enhance survival and to reduce the demand for long-term care.

Disuse atrophy is a type of muscle atrophy, or muscle wasting, which is a decrease in the size of muscles in the body. Disuse atrophy can occur when a muscle is no longer as active as usual. Muscles that are no longer in use will slowly become weaker and eventually, they begin to shrink. In some cases, disuse atrophy can be reversed if the muscles become active again. Disuse atrophy can be caused by immobility, such as an arm being in a cast for a long period of time. It can also occur to some degree if a person stops their usual activities, such as walking.

We have previously shown that administration of GDF5 can improve sarcopenia or disuses atrophy. In this respect, reference is made to WO2020016425 and Traoré et al., Sci Transl Med., 2019, Nov 6;11(517).

In some embodiments, the GDF5-PEG protein is administered to rejuvenate muscle is a subject having sarcopenia or disuse atrophy. In other embodiment, the GDF5-PEG protein is administered to improve muscle function is a subject having sarcopenia or disuse atrophy. In other embodiment, the GDF5-PEG protein is administered to improve muscle mass is a subject having sarcopenia or disuse atrophy. In some embodiments, the GDF5-PEG protein is administered to improve muscle strength is a subject having sarcopenia or disuse atrophy. In yet other embodiments, the GDF5-PEG protein is administered to increase physical performance or mobility is a subject having sarcopenia or disuse atrophy.

Other non-limiting benefits of the GDF5-PEG protein disclosed herein may include an increase or stabilization of muscle mass and/or function, an increase or stabilization of physical performance or mobility, a decrease of the period of hospitalization, a gain of autonomy, the prevention of risks of death associated to sarcopenia, prevention of cancer mortality and/or the treatment or prevention of frailty in the subject.

In a particular embodiment, the GDF5-PEG protein is to be administered to a subject aged 50 years or older. In particular embodiments, the subject is aged 55 years or older, in particular 60 years or older, more particularly 65 years or older, even more particularly 70 years or older, such as 75 years or older or even 80 years or older. In a particular embodiment, the subject displays progressive muscle mass loss. The subject may be a man or woman, in particular a post-menopausal woman. In a particular embodiment, the subject's motor neurons are intact or substantially intact, meaning that no denervation occurs in the subject's body.

The subject may be screened as suffering, or potentially suffering, from sarcopenia by any means known to those skilled in the art. These include evaluation by calculating skeletal muscle mass index by dual energy X-ray absorptiometry (DEXA) and/or by calculating the body mass index of the subject. Other means for identifying the subject who would benefit from the present invention include the measure of muscle functional parameters.

The invention may also benefit to a subject of the age mentioned above, when the subject undergoes partial or complete body immobilization, such as the immobilization of one limb, such as immobilization of an arm, a leg, a shoulder, a hip, in particular after a fracture of said limb, most particularly a fracture of hip. For example, the immobilization may be the consequence of a bone fracture, such as a fracture of an arm bone, a leg bone, or a fracture of the hip. The immobilization may also follow the replacement of a body part with an artificial part, such as with a prosthesis, a partial or total knee prosthesis, a partial or total hip prosthesis and a partial or total shoulder joint. In a particular embodiment, the GDF5-PEG protein is administered to an aged subject with a hip fracture. The disclosure thus also relates to a GDF5-PEG protein, for use in a method for the treatment or prevention of age-related muscle mass loss in a subject having a partial or complete body immobilization. In particular, it is herein disclosed a GDF5-PEG protein, for use in a method for the treatment or prevention of age-related muscle mass loss in a subject having a hip fracture.

The GDF5-PEG protein may also benefit to subjects suffering from muscle mass loss resulting from microgravity.

The GDF5-PEG protein may also benefit to subjects suffering from progeria. Progeria, also known as Hutchinson-Gilford syndrome, is an extremely rare, progressive genetic disorder that causes children to age rapidly, starting in their first two years of life. Children with progeria generally appear normal at birth. During the first year, signs and symptoms, such as slow growth and hair loss, begin to appear. A loss of muscle mass is also observed in the children suffering from this disease. Accordingly, the GDF5-PEG protein may be beneficial for subjects suffering from progeria, by at least alleviating one of the symptoms of the disease. As such, in some embodiments the GDF5-PEG protein is for use in a method for treating or preventing progeria-related muscle mass loss, increase or stabilizing muscle mass and/or function, or for increasing or stabilizing physical performance or mobility of a subject suffering from progeria.

In another aspect, the GDF5-PEG protein may also be administered to treat or prevent disuse atrophy in a subject in need thereof. In this aspect, the subject may be either young or old. For example, the disuse atrophy may be the result, or the result-to-be, of a partial or complete body immobilization, such as the immobilization of a limb, for example the immobilization of an arm, a leg, a shoulder, or a hip. For example, the immobilization may be the consequence of a bone fracture, such as a fracture of an arm bone, a leg bone, or a fracture of the hip. The immobilization may also follow the replacement of a body part with an artificial part, such as with a prosthesis, a partial or total knee prosthesis, a partial or total hip prosthesis and a partial or total shoulder joint replacement. The immobilization may also be the consequence of the subject being in a coma state. As such the GDF5-PEG protein may be beneficial in that it may treat or prevent muscle wasting observed during a coma episode. For example, the GDF5-PEG protein may be beneficial to a subject in a coma state to increase or stabilize muscle mass and/or function, to increase or stabilize physical performance or mobility, or to prevent or treat coma-associated frailty in the subject.

In another particular aspect, the present disclosure provides the use of a GDF5-PEG protein for obtaining a non-therapeutic muscle mass and/or strength gain. For example, such muscle mass and/or strength gain may be desired in the context of sport practice or exercise. In this embodiment, the subject may be a young subject, for example a teenager or a young adult, such as a subject aged from 11 to 50 year, in particular from 15 to 40 year, such as from 18 to 30 year.

In another aspect, the GDF5-PEG protein can be used in a method for the treatment of muscle weakness in a subject suffering from myopathy or from a neuromuscular disorder. Weakness is one of the predominant clinical manifestations of myopathies and neuromuscular diseases, which strongly influences daily life, prognosis, and outcome of affected patients. One of the major therapeutic goals in subjects suffering from these diseases is to completely resolve muscle weakness. Various treatment options are available and include physical therapy, electrotherapy, diet, drugs, avoidance or withdrawal of muscle-toxic and weakness-inducing agents, detoxification, stem-cell-therapy, plasma-exchange, respiratory therapy, or surgery. Thanks to the GDF5-PEG protein disclosed herein, muscle weakness may be treated or prevented by administration of said protein to a subject in need thereof. The GDF5-PEG protein may be administered alone, or in combination with a treatment for a myopathy or neuromuscular disease. Therefore, it is also herein disclosed a GDF5-PEG protein for use in combination with a treatment for a myopathy or neuromuscular disease, such as in combination with another pharmaceutically active substance suitable for the treatment of said condition. Thanks to the GDF5-PEG protein, great benefits may be obtained in the context of such treatments for a myopathy or a neuromuscular disease, such as an increase or stabilization of muscle mass and/or function or an increase or stabilization of physical performance or mobility, thereby synergistically increasing the therapeutic efficiency of said treatment against a myopathy or neuromuscular disease. It is also described herein a GDF5-PEG protein for use in increasing or stabilizing the muscle mass and/or function or increasing or stabilizing physical performance or mobility in a subject receiving a treatment for a myopathy or neuromuscular disease, such as a subject suffering from a myopathy such as a centronuclear myopathies and dystrophinopathies (e.g. Duchenne muscular dystrophy or Becker muscular dystrophy) or from a neuromuscular disease such as spinal muscular atrophy and amyotrophic lateral sclerosis. In some aspects, the motor neurons of the subject are intact or substantially intact.

Another aspect relates to the treatment of a muscular disease mediated by an affection of motor neurons. Indeed, it was previously shown that the GDF5 pathway leads to the activation of a compensatory pathway in atrophic or sarcopenic muscles. Therefore, it is possible to compensate the lack of innervation or impaired innervation thanks to the GDF5-PEG protein disclosed herein. Muscular diseases that could be treated thanks to the invention include, without limitation, myopathies such as centronuclear myopathies and dystrophinopathies (e.g. Duchenne muscular dystrophy or Becker muscular dystrophy), neuromuscular diseases such as spinal muscular atrophy and amyotrophic lateral sclerosis, and congenital and traumatic spinal cord injury.

The present disclosure further provides the GDF-PEG protein described herein, for use in a method for the treatment of a demyelinating neuropathy disease involving Schwann cells, such as Charcot-Marie-Tooth disease and Guillain-Barré syndrome (acute inflammatory demyelinating polyradiculopathy type).

The present disclosure also provides the GDF5-PEG protein described herein, for use in a method for the treatment of a muscular disease, in combination with another active ingredient. Said other active ingredient is suitable for the treatment of the muscular disease. Such active ingredients include, without limitation:
- an antisense oligonucleotide (AON) suitable for exon-skipping within the SOD 1 pre-mRNA, for the treatment of amyotrophic lateral sclerosis;
- a vector, such as a viral vector, in particular an AAV vector, comprising a gene encoding a SMN protein, such as the SMN 1 or SMN2 gene, more particularly the SMN 1 gene, for the treatment of spinal muscular atrophy;
- a vector, such as a viral vector, in particular an AAV vector, comprising a gene encoding a functional dystrophin, for the treatment of Duchenne muscular dystrophy or Becker muscular dystrophy; or
- (i) an AON suitable for exon-skipping within the dystrophin pre-mRNA, or a vector encoding the same, and (ii) a vector, such as a viral vector, in particular an AAV vector, comprising a gene encoding a functional dystrophin, for the treatment of Duchenne muscular dystrophy.

The active ingredients, i.e. the GDF5-PEG protein and the other active ingredient suitable for treatment of a muscular disease, can be for sequential, separate or simultaneous use. In some embodiments, the GDF5-PEG protein may be administered before, during, or after the administration of a gene therapy vector or of an AON. In some embodiments, the GDF5-PEG protein may be administered before administration of an AON, such as an AON for use in the treatment of amyotrophic lateral sclerosis, Duchenne muscular dystrophy or Becker muscular dystrophy. In some embodiments, the GDF5-PEG protein may be administered during administration of an AON, such as an AON for use in the treatment of amyotrophic lateral sclerosis, Duchenne muscular dystrophy or Becker muscular dystrophy. In this embodiment, the GDF5-PEG protein and the AON may be either in the same composition, or in different compositions. In another example, the GDF5-PEG protein may be administered after administration of an AON, such as an AON for use in the treatment of amyotrophic lateral sclerosis, Duchenne muscular dystrophy or Becker muscular dystrophy. According to another embodiment, the GDF5-PEG protein may be administered before administration of a gene therapy vector for use in the treatment of a muscular disease, such as a gene therapy vector for the treatment of spinal muscular atrophy, Duchenne muscular atrophy or Becker muscular atrophy. In a particular embodiment, the GDF5-PEG protein may be administered during administration of a gene therapy vector, such as a gene therapy vector for use in the treatment of a muscular disease, such as a gene therapy vector for the treatment of spinal muscular atrophy, Duchenne muscular atrophy or Becker muscular atrophy. In this embodiment, the GDF5-PEG protein and the gene therapy vector may be either in the same composition, or in different compositions. In another particular embodiment, the GDF5-PEG protein may be administered after administration of a gene therapy vector for use in the treatment of a muscular disease, such as a gene therapy vector for the treatment of spinal muscular atrophy, Duchenne muscular atrophy or Becker muscular atrophy.

Of course, the GDF5-PEG protein disclosed herein may also have applications and benefits in the veterinary field. In particular, any embodiment described above may be implemented in a non-human mammalian such as pets and livestock. In particular, the GDF5-PEG protein may be administered to a pet to treat or prevent sarcopenia in said pet or livestock, in particular in a pet. In particular, the GDF5-PEG protein may be administered to a pet to increase or stabilize muscle mass and/or function, to increase or stabilize physical performance or mobility, to decrease the period of hospitalization in a veterinary hospital, to increase gain of autonomy, to prevent the risks of death associated to sarcopenia, to prevent cancer mortality and/or to treat or prevent frailty in said pet. According to a particular embodiment, a pet includes, without limitation, any non-human mammalian that may be kept for a person's company. These include cats, dogs, rabbits, rats, mice, hamsters, guinea pigs, ferrets, horses and pigs, without limitation. In a particular embodiment, the pet is a cat or a dog.

In another aspect, the present disclosure relates to a non-therapeutic method for increasing muscle mass and/or function in a non-human animal, in particular in livestock, the method comprising administering to said animal a GDF5-PEG protein in an amount effective to induce and increase in muscle mass and/or function. In the context of the present invention, livestock are domesticated animals raised in an agricultural setting to produce labor and/or commodities such as meat, eggs, milk, fur, leather, and wool. The term includes, without limitation, cattle, pigs, sheep, goats and horses.

Of course, in all non-human mammal aspects described above, the GDF5-PEG protein specifically used will be selected according to the specific non-human mammalian receiving said substance. For example, it may be more suitable to use a GDF5 protein moiety encoding the peptide or protein derived from said non-human mammal. As an illustration, a cat, dog, rabbit, rat, mouse, hamster, guinea pig, ferret, horse, pig, cattle, sheep, goat and horse GDF5 protein moiety may be preferably used in a cat, dog, rabbit, rat, mouse, hamster, guinea pig, ferret, horse, pig, cattle, sheep, goat and horse subject, respectively.

### EXAMPLES

### Example 1: materials and methods

### Animals

Adult (2 months) C57/BL6 female mice were purchased from Janvier Lab and old (20 months) C57/BL6 female mice were purchased from Charles River Lab. Mice were housed at the animal facility under specific-pathogen-free (SPF) conditions, under 12 h/12h light/dark conditions, with ad libitum access to food and water. Animals were acclimated for a minimum of 2 weeks before being used for experiments.

### Synthetic GDF5 administration

A solution of vehicle containing 0.1% bovine serum albumin (BSA) diluted in PBS or chemically synthesized GDF5 candidates diluted in vehicle at a dose of 0.2 mg/kg was injected subcutaneously (over the shoulders, into the loose skin over the neck). GDF5 candidates were injected in 2-month-old-mice once for stability and activity studies. For functional evaluations, GDF5 candidates were injected at 0.2 mg/kg in 20-month-old mice twice per week (every three days) for four months.

### Immunofluorescence

For immunofluorescence procedures, 10 µm-thick *Tibialis Anterior* (TA) muscle cryosections were fixed in 4% paraformaldehyde (PFA) (Sigma, 1.00496.0700), permeabilized with 0.5% Triton X-100 (Merck Millipore) and blocked in 10% Horse serum (HS), 0.2% Triton, 5% BSA-PBS. Muscle cryosections were incubated overnight at 4°C with the following primary antibodies diluted in blocking solution: anti-p-SMAD 1/5 (S463/465 - cell signaling, 9516S, 1/800), anti-laminin (Santa Cruz, sc-59854, 1/50). After washes in PBS, sections were incubated with the following secondary antibodies for 1 hour at room temperature: Alexa Fluor 546 goat anti-rabbit IgG (H+L) (Invitrogen, A11071, 1/500) and Alexa Fluor 488 donkey anti-rat IgG (H+L) (Life Technologies, A21208, 1/500). Staining of nuclei was performed using DAPI (Invitrogen, D21490, 1/5000). Slides were mounted with Fluoromont-G^{™} Medium (Invitrogen).

### Image acquisition

Fluorescence images were acquired using a Spinning Disk confocal scanner unit (CSU-W1 YOKOGAWA) with a 40x oil-immersion objective (plan fluor 40x/1.3 oil OFN25 DIC N2 cyan), coupled with a Photometrics PRIM 95B Camera. Software used was MetaMorph 7.10.2. Images were merged and edited in ImageJ (version 1.53e).

In details, four fields of each muscle section were acquired using 40x oil objective. The images analyzed are projected images obtained by overlying the collected z-stacks, in order to evaluate all the nuclear signal present in the muscle section thickness. p-SMAD 1/5 nuclear signal was analyzed by using ImageJ macro developed in the lab in collaboration with MyoImage facility.

### Activation of SMAD 1/5 pathway quantification by application of normal distribution

The probability of activation of the SMAD 1/5 pathway in Tibialis Anteriors (TAs) (n=3-6 mice per each treatment condition and 10 mice for vehicle group) was quantified by measuring the mean (µ) and standard deviation (σ) of the fluorescence intensities ofp-SMAD 1/5 in all nuclei (~350-400 nuclei/TA). From µ and σ values, the normal distribution was applied to calculate the probability of activation according to the Excel formula: = NORMDIST(X; µ; σ; FALSE). Then, for each TA, the µ*2σ formula was used to estimate the area under the curve with a 95% confidence interval. The values obtained from the estimated area were normalized to the vehicle group values.

### Grip strength measurement

We used a grip strength meter (Bioseb) to assess hindlimbs and forelimbs grip strength. Mice were lifted and held by their tail such that their forepaws could grasp a wire grid; subsequently, they were gently pulled backwards until they released the grid. Each mouse was subjected to six consecutive tests and peak force was registered with 30 seconds rest period. To minimize variability in performance, the minimal and maximal values of grip strength were removed for each animal (mean= 4 values). Mean of grip strength was determined in grams (g) and normalized to the body weight. Grip strength experiments were performed in a blinded manner by the same investigator.

### Electroneuromyography measurements

Electroneuromyography (ENMG) measurements were achieved in TA muscle. Two monopolar reference and recording electrodes were inserted, one close to the patella tendon and the other in the middle of the TA muscle. A monopolar ground electrode was also inserted into the contralateral hindlimb muscle. The sciatic nerve was stimulated with series of 10 stimuli at 20 Hz. Compound muscle action potentials (CMAP) were amplified (BioAmp, ADInstruments), acquired with a sampling rate of 100 kHz, filtered with a 5 kHz low-pass and a 1 Hz high-pass filter (Powerlab 8/25, ADInstruments). Peak-to-peak amplitudes were analyzed with LabChart 8 software (ADInstruments). During ENMG experiments mouse body temperature was maintained at 37°C with radiant heat. ENMG experiments were performed in a blinded manner.

### TA muscle contraction measurement

The function of TA muscles was evaluated by measuring *in vivo* muscle contraction in response to nerve stimulation. Mice were anesthetized using a mix of ketamine (100mg/ml) and xylazine (20 mg/ml) diluted in NaCl 0.9% solution. Ketamine/Xylazine solution was injected intraperitoneally (100µl/mouse 20g). At the same time the mice were injected subcutaneously with 0.1 mg/Kg of buprenorphine (Vetergesic) resuspended in a 0.9% NaCl solution using a 29G insulin syringe. The knee and paw were fixed in place, and the distal tendon of the muscle was attached to the lever arm of a servomotor system (305B, Dual-Mode Lever, Aurora Scientific) using a silk ligature.

Capacity for force generation was evaluated during isometric contractions in response to sciatic nerve electrical stimulation (frequency of 1Hz; train of stimulation of 500 ms). Time to peak was determined in ms. Data were analyzed using the PowerLab system (4SP, ADInstruments) and software (Chart 4, ADInstruments).

After measurements, mice were sacrificed by cervical dislocation under general anesthesia and muscles were dissected, weighted and frozen in isopentane precooled in liquid nitrogen and stored at -80 °C for histological analysis. Measurements were performed in a blinded manner.

### Statistical analyses

Before statistical analyses, a preliminary Shapiro-Wilk test was performed. For comparison between two groups non parametric T-test was used. For comparison among more than two groups, ordinary one way ANOVA test was performed followed by appropriated post hoc multiple comparison Tukey's HSD or Fisher's LSD tests. P-value: * <0.05; **<0.01; ***< 0.001.

All these analyses were performed using Prism GraphPad 9 software.

### Example 2: results

In order to try to identify GDF5 for clinical use, we have carried out a number of modifications of the protein. One of the design implemented was to chemically synthesized human GDF5, and then to modify the chemically synthesized protein by addition of different PEG polymers. In the study summarized below, we report the results obtained with GDF5 linked to linear PEG having a molecular weight of 20,000 Da (GDF5-PEG-20,000), or with branched PEG with a molecular weight of 2 x 10,000 Da (GDFS-PEG-2x10.000) or of 2 x 20,000 Da (GDF5-PEG-2x20.000).

We conducted a short-term study to assess product efficacy. For this study, we used young female mice injected 1 time at a dose of 0.2 mg/kg subcutaneously. We compared activation of the GDF5 pathway, measured by phosphorylation of SMAD 1/5 proteins in the nuclei of TA muscle fibers (Traoré et al. Sci Transl Med. 2019), by modified human GDF5 candidates and unmodified human GDF5 protein (native GDF5) respectively, to vehicle (PBS-0.1%BSA). We chose to assess this activation 48h after injection because preliminary data collected in the laboratory showed maximal activation of the GDF5 pathway 48h after injection of the recombinant murine, commercial protein.

All TAs fiber nuclei from treated mice were assessed for their labeling intensity of phosphorylated SMAD1/5 proteins. Data were analyzed using the normal distribution with values of nuclei intensities on the abscissa and probability of having this intensity on the ordinate. Once the values were normally distributed, we calculated the positive portion of the area under the curve. This area was expressed as a relative value to the positive area under the curve of the vehicle group. This is shown on the ordinate in Figure 1. The results of the comparative study of the 4 products and the vehicle showed that GDF5-PEG20.000 was the candidate that activated the SMAD1/5 pathway more effectively than native GDF5 and the other candidates (Figure 1).

We also evaluated the stability of the products by analyzing the activation of the GDF5 pathway for 7 days after the injection of the different molecules (Figure 2). For this study, we used young female mice injected 1 time at a dose of 0.2 mg/kg subcutaneously. Specifically, our results showed that 7 days after injection, human GDF5-PEG20,000 still induced activation of the SMAD1/5 pathway while this was not the case for native GDF5 (Figure 2). In addition, although there was a better trend for GDF5-PEG20,000, there was no significant difference in the stability of this product compared to other modified human GDF5. This result suggests that GDF5-PEG20,000 has a stable activity over time compared to native human GDF5 and a better SMAD1/5 pathway activation kinetics compared to other candidates.

Taken together, these results show that GDF5-PEG20,000 induces activation of the SMAD1/5 pathway faster and more efficiently compared to native GDF5 and GDFS-PEGY2x10,000 and GDF5-PEGY2x20,000 (Figure 1). In addition, the results show that over time GDF5-PEG20,000 has better stability than native GDF5 (Figure 2).

We then assessed the GDF5-PEG20,000 protein in a long-term study for its functional effects, namely as a trophic factor in muscle mass. Notably, the improvement in grasping activity and nerve-muscle connectivity was compared to that induced by the native human GDF5 protein. For this study, 20-month-old mice were injected 2 times per week subcutaneously for 16 weeks at a dose of 0.2 mg/kg (Figure 3). The data showed that after GDF5-PEG20,000 injections, the mass of the TA was significantly increased and the force (measured by the grip strength test) was significantly greater compared to the condition with native GDF5 and increases as compared to the vehicle (Figure 3A-B). In addition, the native human GDF5 protein did not induce variations in muscle mass and/or strength during this protocol. The decrement value of the action potential measured at the end of the protocol (Final) and compared to the value measured before the start of the injections (Baseline) showed that the GDF5-PEG20,000 treatment allowed a significant improvement in neuromuscular transmission, which was not the case for the native human GDF5 during this protocol (Figure 3C). Finally, we observed a 10% decrease in the time of muscle contraction in muscles treated with GDF5-PEG20,000 revealing an improvement in nerve-muscle connectivity (Figure 3D).

In conclusion, thanks to these studies we have shown an unexpected advantage to the optimization of the human GDF5 protein by the addition of a linear polyethylene glycol polymer with a molar mass of 20,000 g/mol, compared to the other branched optimized human forms (GDF5-PEG Y-2x10000 and GDF5-PEG Y-2x20000) and to the unmodified human GDF5 protein.

## Claims

1. A pegylated Growth Differentiation Factor 5 (GDF5-PEG) protein.

2. The GDF5-PEG protein according to claim 1, wherein the GDF5 protein moiety is a chemically synthesized protein.

3. The GDF5-PEG protein according to claim 1 or 2, wherein the GDF5 protein moiety is human GDF5.

4. The GDF5-PEG protein according to claim 3, wherein the human GDF5 protein comprises or consists of the amino acid sequence shown in SEQ ID NO: 1, or comprises or consists of a sequence which is at least 90% identical to SEQ ID NO:1, or at least 91%, at least 92%, at least 93%, at least 94%, at least 95%, at least 96%, at least 97%, at least 98% or at least 99% identical to SEQ ID NO: 1

5. The GDF5-PEG protein according to any one of claims 1 to 4, wherein the GDF5-PEG protein comprises a linear PEG molecule having a molecular weight of from 5,000 g/mol to 50,000 g/mol.

6. The GDF5-PEG protein according to any one of claims 1 to 5, wherein the GDF5-PEG protein comprises a linear PEG molecule having a molecular weight of from 10,000 g/mol to 30,000 g/mol.

7. The GDF5-PEG protein according to any one of claims 1 to 6, wherein the GDF5-PEG protein comprises a linear PEG molecule having a molecular weight of 20,000 g/mol.

8. The GDF5-PEG protein according to any one of claims 1 to 7, for use in a method for the treatment or prevention of sarcopenia or disuse atrophy.

9. The GDF5-PEG protein according to any one of claims 1 to 7, for use in treating or preventing muscle weakness in a myopathy or neuromuscular disease, alone or in combination with a treatment of said myopathy or neuromuscular disease.

10. The GDF5-PEG protein according to any one of claims 1 to 7, for use in the treatment of a muscular disease, in combination with at least one other active ingredient suitable for use in the treatment of said muscular disease.

11. The GDF5-PEG protein for use according to claim 10, wherein the at least one other active ingredient is:
i. the combination of (i) an antisense oligonucleotide (AON) capable of inducing an exon-skipping in a dystrophin pre-mRNA, and (ii) a viral vector, such as an AAV vector, encoding a Duchenne muscular dystrophy therapeutic product, for use in the treatment of Duchenne muscular dystrophy;
ii. an antisense oligonucleotide (AON) capable of inducing an exon-skipping in the SOD1 pre-mRNA, thereby leading to the incorporation of a premature stop codon into the mature mRNA, for use in the treatment of amyotrophic lateral sclerosis; or
iii. a vector comprising a gene encoding a survival of motor neuron protein, such as the SMN 1 or SMN2 gene.
